# EUROPEAN PATENT SPECIFICATION

(11) **EP 2 630 948 B1**
(45) Date of publication and mention of the grant of the patent: **22.08.2018**
(21) Application number: 11834137.9
(22) Date of filing: 13.09.2011
(51) Int. Cl.: A61K 8/81, A61K 8/06, A61K 8/39, A61K 8/46, A61K 8/893, A61Q 1/02, A61Q 1/10, A61Q 1/14, A61Q 17/04, A61Q 19/00, A61Q 19/02

(54) **EXTERNAL OIL-IN-WATER-TYPE SKIN PREPARATION**
EXTERNES ÖL-IN-WASSER-PRÄPARAT FÜR DIE HAUT
PRÉPARATION EXTERNE DE TYPE HUILE-DANS-EAU POUR LES SOINS DE LA PEAU

(30) Priority: 19.10.2010 JP 2010234335
(43) Date of publication of application: 28.08.2013
(73) Proprietor: SHISEIDO COMPANY, LTD., Tokyo 104-0061 (JP)
(72) Inventor: HIRUMA, Takuya, Yokohama-shi Kanagawa 224-8558 (JP); KANEMARU, Tetsuya, Yokohama-shi Kanagawa 224-8558 (JP); SATO, Yukiko, Yokohama-shi Kanagawa 224-8558 (JP); KUBOTA, Shun, Yokohama-shi Kanagawa 224-8558 (JP); YAGI, Katsuhiko, Yokohama-shi Kanagawa 224-8558 (JP)
(74) Representative: Lippert Stachow Patentanwälte Rechtsanwälte
(86) International application number: PCT/JP2011/070833
(87) International publication number: WO 2012/053295

(56) References cited:
- EP-A1- 1 369 102
- JP-A- 2009 256 625
- SHIGERU SEKINE KESHOHIN HANDBOOK 01 November 1996, pages 126 - 133, XP008170781

## Description

### RELATED APPLICATIONS

This application claims the priority of Japanese Patent Application No. 2010-234335 filed on October 19, 2010.

### FIELD OF THE INVENTION

The present invention relates to an oil-in-water external skin preparation, and in particular, relates to an oil-in-water external skin preparation having an excellent stability, excellent rough-surface correction effect, and excellent texture.

### BACKGROUND OF THE INVENTION

In the past, functions such as the protection of the skin from ultraviolet light, the concealing of pigmented spots, freckles, etc., and the absorption of sebum have been provided by blending powder in the external skin preparation.

In particular, the oil-in-water external skin preparation has good texture and multifunctionality, thus it has been used by blending powder therein; for example, as emulsion-type foundation, milky lotion, and cream.

On the other hand, silica has been the only virtually stable-mixable powder ingredient in the aqueous phase of the oil-in-water external skin preparation, (for example, refer to patent literature 1). However, only a hard powdery texture, which is peculiar to silica, could be provided to the preparation. In addition, the stability often decreased when a large amount was blended.

In recent years, resin particles have been known (for example, refer to patent literature 2). These resin particles have been known to be usable as a mattifying agent for paint, and in optical film, anti-reflection film, external preparations, etc.

Patent literature 1: Japanese unexamined patent publication No. 2004-217534 Patent literature 2: Japanese unexamined patent publication No. 2009-256625 discloses an aqueous medium comprising a vinyl monomer of 100 parts by weight and an aliphatic (meth) acrylate monomer of 0.1 to 20 parts by weight containing a hydroxyl ether group and a hydroxyl group or an ester group and discloses a polymerization initiator method for producing resinous particles, characterized by obtaining the resin particles by polymerizing a monomer mixture.

EP 1369102 A1 discloses an oil-in-water emulsion which comprises mixing together a high molecular compound carrying at least on side chains thereof groups comprising ether bonded subgroups and terminal C4-30 alkyl subgroups, an aqueous solution of a water-soluble polyol and a hydrophobic compound, and diluting a resulting mixture with water.

### DISCLOSURE OF THE INVENTION

### PROBLEM TO BE SOLVED BY THE INVENTION

The texture is expected to be provided by blending the above-described powder in the oil-in-water external skin preparation; however, there is room for the improvement of stability.

The present invention was made in view of the above-described conventional art. An object of the invention is to provide an oil-in-water external skin preparation that has an excellent stability, excellent rough-surface correction effect, and excellent texture.

### MEANS TO SOLVE THE PROBLEM

The present inventors have diligently studied to solve the above-described problem. As a result, the present inventors have found that an oil-in-water external skin preparation with good emulsion stability, an excellent rough-surface correction effect, and excellent texture can be obtained by blending resin particles in the aqueous phase and emulsifying with a specific surfactant according to claim 1, thus leading to the completion of the present invention.

That is, the oil-in-water external skin preparation of the present invention is characterized by comprising (a) an aqueous phase containing resin particles obtained from (i) 100 parts by weight of a vinyl-type monomer as specified in claim 1 and (ii) 0.1 to 20 parts by weight of a monomer represented by the below general formula (I),
(b) an oil phase, and
(c) a surfactant comprising one or more selected from polyoxyethylene hydrogenated castor oil, silicone-type surfactants, and sulfonic acid-type surfactants, in the amount of 0.01 to 5 % by mass of the total amount of the external skin preparation and 30 % by mass or more of the total amount of surfactants,
and wherein the oil-in-water external preparation is obtainable by blending the resin particles in the aqueous phase and emulsifying with a specific surfactant. 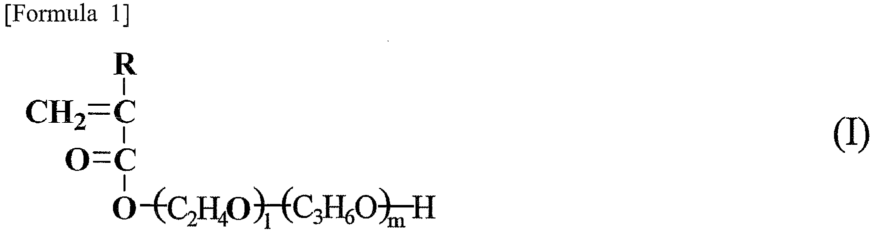 In the above formula (I), R represents a hydrogen atom or a methyl group, 1 represents 1 to 50, and m represents 1 to 50.

In the above external skin preparation, it is preferred that the above-described resin particles are contained in the amount of 0.1 to 13 % by mass of the total amount of the external skin preparation.

Furthermore, in the above external skin preparation, it is preferred that the powder dispersed in the oil phase is contained.

### EFFECT OF THE INVENTION

The oil-in-water external skin preparation of the present invention comprises (a) an aqueous phase containing resin particles, (b) an oil phase, and (c) a surfactant comprising one or more selected from polyoxyethylene hydrogenated castor oil, silicone-type surfactants, and sulfonic acid-type surfactants. Accordingly, the oil-in-water external skin preparation excellent in stability and also excellent in the rough-surface correction effect and texture can be provided.

### BRIEF DESCRIPTION OF THE DRAWINGS

Fig. 1 shows adsorption isotherms for the surfactant (polyoxyethylene (60) hydrogenated castor oil) to represent the adsorption characteristics of the resin particles and spherical silica.
Fig. 2 shows adsorption isotherms for the respective surfactants to represent the adsorption characteristics of the resin particles and spherical silica.

### BEST MODE FOR CARRYING OUT THE INVENTION

The oil-in-water external skin preparation of the present invention consists of (a) an aqueous phase containing resin particles, (b) an oil phase, and (c) a surfactant comprising one or more selected from polyoxyethylene hydrogenated castor oil, silicone-type surfactants, and sulfonic acid-type surfactants, and the further features of claim 1. In the following, each component is described in detail.

### ((a) Aqueous phase containing resin particles)

In the oil-in-water external skin preparation of the present invention, it is necessary that the resin particles are contained in the aqueous phase. The resin particles are polymers obtained from (i) 100 parts by weight of a vinyl-type monomer as further specified in claim 1 and (ii) 0.1 to 20 parts by weight of a monomer represented by the below general formula (I).

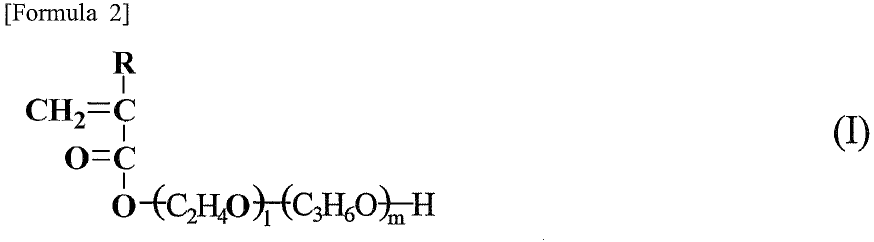

In the general formula (I), R represents a hydrogen atom or a methyl group. 1 represents 1 to 50 and m represents 1 to 50. If 1 exceeds 50, the polymerization stability is lowered and fused particles tend to be generated. If m exceeds 50, the polymerization stability is lowered and fused particles tend to be generated.

According to claim 1 vinyl-type monomers of component (i) include acrylic acids or their esters such as acrylic acid, methyl acrylate, ethyl acrylate, n-butyl acrylate, isobutyl acrylate, and 2-ethylhexyl acrylate, and methacrylic acids or their esters such as methacrylic acid, n-butyl methacrylate, 2-ethylhexyl methacrylate, methyl methacrylate, ethyl methacrylate, isobutyl methacrylate, cyclohexyl methacrylate, benzyl methacrylate, isobornyl methacrylate, 2-hydroxyethyl methacrylate, 2-ethoxyethyl methacrylate, glycidyl methacrylate, tetrahydrofurfuryl methacrylate, diethylaminoethyl methacrylate, trifluoroethyl methacrylate, and heptadecafluorodecyl methacrylate.

According to claim 1 vinyl-type monomers of component (i), other than (meth)acrylic acid-type monomers, include those containing a vinyl group, such as styrene, p-methylstyrene, α-methylstyrene, and vinyl acetate.

The above-described vinyl-type monomers can be used alone or in combination of two or more.

In addition, crosslinked monomers may be contained among the vinyl-type monomers of component (i). Examples of crosslinked monomers include (meth)acrylic acid ester-type monomers such as ethylene glycol di(meth)aclylate, diethylene glycol di(meth)aclylate, triethylene glycol di(meth)aclylate, tetradecaethylene glycol di(meth)aclylate, nonaethylene glycol di(meth)aclylate, tetradecaethylene glycol di(meth)aclylate, decaethylene glycol di(meth)aclylate, pentadecaethylene glycol di(meth)aclylate, 1,3-butylene glycol di(meth)aclylate, 1,4-butanediol di(meth)aclylate, 1,6-hexanediol di(meth)aclylate, glycerin di(meth)aclylate, trimethylol propane tri(meth)aclylate, pentaerithrityl tetra(meth)aclylate, diethylene glycol phthalate di(meth)aclylate, caprolactone-modified dipentaerithrityl hexa(meth)aclylate, caprolactone-modified hydroxypivalate ester neopentyl glycol diaclylate, polyester aclylate, and urethane aclylate, and aromatic divinyl-type monomers such as divinyl benzene, divinyl naphthalene, and their derivatives. These crosslinked monomers can be used alone or in combination of two or more.

The amount of the crosslinked monomers is preferably 0.5 to 80% by weight of the total amount of vinyl-type monomer and more preferably 5 to 50% by weight. If the percentage of crosslinked monomers is too high, the powder may become hard. If the percentage of crosslinked monomers is too low, the resistance to solvents may become low.

The production method of the resin particles of the present invention is not limited in particular, and publicly known methods such as suspension polymerization, emulsion polymerization, and seed polymerization can be listed.

The suspension polymerization and emulsion polymerization are not limited in particular, and they can be carried out by suspending or emulsifying a mixture of monomers under publicly known conditions and subsequent polymerization. A monomer represented by the general formula (I) may be dissolved in a vinyl-type monomer or dispersed in an aqueous medium, and a publicly known suspension stabilizer or emulsifier may also be used.

The resin particles, which are blended in the aqueous phase, of the present invention, are contained in the amount of 0.1 to 13 % by mass of the total amount of the external skin preparation, preferably in the amount of 0.5 to 10 % by mass, and more preferably in the amount of 1 to 8 % by mass. If the blending quantity of the resin particles is less than 0.1 % by mass, the blending effect cannot be expected to be realized. If the blending quantity of the resin particles exceed 13 % by mass, the stability over time may be poor.

In the (a) aqueous phase, in addition to the above-described essential component, namely resin particles, aqueous components normally usable in cosmetics, pharmaceuticals, etc. can be blended within the range that the emulsion stability is not impaired.

It is preferred that water is blended as a main component of (a) water phase. Also, it is preferred that lower alcohol such as ethanol is blended.

Further, it is preferred that one or more thickener(s) selected from succinoglycan, xanthan gum, and acrylamide is blended in (a) water phase. By blending thickener(s), the dispersion stability of powder can be further improved. In addition, an improvement can be made in the time-dependent settling and creaming of emulsion particles.

Further, it is also preferred that one or more coemulsifier(s) selected from carboxymethyl cellulose, hydroxyethyl cellulose, hydroxymethyl cellulose, and gelatin is blended.

Further, examples of moisturizers which can be blended in the water phase include 1,3-butylene glycol, polyethylene glycol, propylene glycol, dipropylene glycol, hexylene glycol, glycerin, diglycerin, xylitol, maltitol, maltose, and D-mannite.

Examples of water-soluble polymers include plant-type polymers such as gum arabic, carrageenan, pectine, agar, quince seed (marmelo), and algae colloid (brown algae extract), microorganism-type polymers such as dextran and pullulan, animal-type polymers such as collagen, casein, and gelatine, starch-type polymers such as starch, carboxymethyl starch, and methylhydroxypropyl starch, alginic acid-type polymers such as sodium alginate, vinyl-type polymers such as carboxyvinyl polymer (e.g., CARBOPOL®), polyoxyethylene-type polymers, polyoxyethylene/polyoxypropylene copolymer-type polymers, acrylic-type polymers such as sodium polyacrylate and polyacrylamide, and inorganic-type water-soluble polymers such as bentonite, magnesium aluminium silicate, and laponite.

Examples of UV absorbers include benzoic acid-type UV absorbers such as p-aminobenzoic acid, anthranilic acid-type UV absorbers such as methyl anthranilate, salicylic acid-type UV absorbers such as octyl salicylate and phenyl salicylate, cinnamic acid-type UV absorbers such as isopropyl p-methoxycinnamate, octyl p-methoxycinnamate, and glyceryl mono-2-ethylhexanoate di-p-methoxycinnamate, benzophenone-type UV absorbers such as 2,4-dihydroxybenzophenone, 2-hydroxy-4-methoxybenzophenone, and 2-hydroxy-4-methoxybenzophenone-5-sulfonic acid, urocanic acid, 2-(2'-hydroxy-5'-methylphenyl)benzotriazole, and 4-tert-butyl-4'-methoxybenzoylmethane.

Examples of sequestering agents include sodium edetate, sodium metaphosphate, and phosphoric acid.

Examples of antioxidants include ascorbic acid, α-tocopherol, dibutyl hydroxytoluene, and butylated hydroxyanisole.

Examples of drugs include vitamins such as vitamin A, retinol, retinol palmitate, inositol, pyridoxine hydrochloride, benzyl nicotinate, nicotinamide, dl-α-tocopherol nicotinate, magnesium ascorbyl phosphate, ascorbic acid 2-glucoside, vitamin D2 (ergocalciferol), L-ascorbic acid dl-α-tocopherol phosphoric acid diester potassium salt, dl-α-tocopherol, dl-α-tocopheryl acetate, pantothenic acid, and biotin, anti-inflammatory agents such as allantoin and azulene, whitening agents such as arbutin, astringent agents such as zinc oxide and tannic acid, sulfur, lysozyme chloride, pyridoxine hydrochloride, and gamma-orizanol.

The above-described drugs can be used in a free state, a form of acid or basic salt if one can become salt, or a form of ester if one has a carboxylic acid group.

The blending quantity of the (a) aqueous phase, namely aqueous components, is preferably 50 to 90 % by mass of the total amount of the external skin preparation, and especially preferably 60 to 80 % by mass.

### ((b) Oil phase)

In the (b) oil phase, oil components normally usable in cosmetics and pharmaceuticals can be blended within the range that the emulsion stability is not impaired.

Examples of liquid oils include silicone oils. Examples of silicone oils include linear silicone oils such as dimethylpolysiloxane, methylphenyl polysiloxane, and methylhydrogen polysiloxane, and cyclic silicone oils such as octamethylcyclotetrasiloxane, decamethylcyclopentasiloxane, and dodecamethylcyclohexasiloxane.

Examples of polar oils include ester oils such as cetyl octanoate, hexyl laurate, isopropyl myristate, octyl palmitate, isocetyl stearate, isopropyl isostearate, octyl isopalmitate, isodecyl isostearate, 2-ethylhexyl succinate, and diethyl sebacate.

Examples of non-polar oils include hydrocarbon oils such as liquid paraffin, squalane, squalene, and paraffin.

Examples of solid oils include solid fats such as cacao butter, coconut oil, horse fat, hydrogenated coconut oil, palm oil, beef fat, mutton suet, and hydrogenated castor oil, hydrocarbons such as paraffin wax (linear hydrocarbon), microcrystalline wax (branched saturated hydrocarbon), ceresin wax, Japan wax, montan wax, and Fischer-Tropsch wax, waxes such as beeswax, lanolin, carnauba wax, candelilla wax, rice bran wax (rice wax), spermaceti, jojoba oil, insect wax, kapok wax, bayberry wax, shellac wax, sugarcane wax, lanolin fatty acid isopropyl, hexyl laurate, reduced lanolin, hard lanolin, POE lanolin alcohol ether, POE lanolin alcohol acetate, POE cholesterol ether, lanolin fatty acid polyethylene glycol, and POE hydrogenated lanolin alcohol ether, higher fatty acids such as myristic acid, palmitic acid, stearic acid, and behenic acid, and higher alcohols such as cetyl alcohol, stearyl alcohol, behenyl alcohol, myristyl alcohol, and cetostearyl alcohols.

The blending quantity of the (b) oil phase, namely oil components, is preferably 1 to 40 % by mass of the total amount of the external skin preparation, and especially preferably 10 to 30 % by mass.

In addition, powder and dispersant may be blended in the oil phase of the present invention. That is, a powder dispersion material containing powder and dispersant can be blended by using the above-described oil component as the dispersion medium. When a powder dispersion material is blended in the oil phase, the stability tends to be poorer than the common oil-in-water emulsion compositions. However, when components (a) to (c) of the present invention are blended, the stability over time is excellent even in the case of an oil-in-water emulsion composition having the oil phase containing powder and dispersant.

The powder and dispersant, which are blended in the oil phase, are not specifically limited and they normally usable in cosmetics and pharmaceuticals can be blended.

Examples of powders include inorganic powder (for example, talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, tungstate, magnesium, silica, zeolite, barium sulfate, calcined calcium sulfate, calcium phosphate, fluorine apatite, hydroxyapatite, ceramic powder, metallic soap (for example, zinc myristate, calcium palimitate, and aluminum stearate), and boron nitride); organic powder (for example, polyamide resin powder (nylon powder), polyethylene powder, polymethylmethacrylate powder, polystyrene powder, styrene-acrylic acid copolymer resin powder, benzoguanamine resin powder, poly(tetrafluroethylene) powder, and cellulose powder); inorganic white family pigment (for example, titanium dioxide and zinc oxide); inorganic red family pigment (for example, iron oxide (colcothar) and iron titanate); inorganic brown family pigment (for example, gamma-iron oxide); inorganic yellow family pigment (for example, yellow iron oxide and ocher); inorganic black family pigment (for example, black iron oxide and low-dimentional titanium oxide); inorganic purple family pigment (for example, manganese violet and cobalt violet); inorganic green family pigment (for example, chrome oxide, chrome hydroxide, and cobalt titanate); inorganic blue family pigment (for example, ultramarine, and iron blue); pearl pigment (for example, titanium oxide coated mica, titanium oxide coated bismuth oxychloride, titanium oxide coated talc, colored titanium oxide coated mica, bismuth oxychloride, and argentine); metal powder pigment (for example, aluminum powder and copper powder); organic pigment such as zirconium, barium, or aluminum lake (for example, organic pigment such as Red No.201, Red No.202, Red No.204, Red No.205, Red No.220, Red No.226, Red No.228, Red No.405, Orange No.203, Orange No.204, Yellow No.205, Yellow No.401, and Blue No.404, Red No.3, Red No.104, Red No.106, Red No.227, Red No.230, Red No.401, Red No.505, Orange No.205, Yellow No.4, Yellow No.5, Yellow No.202, Yellow No.203, Green No.3, and Blue No.1); and natural pigment (for example, chlorophyll and β-carotene).

When powder is blended, it is preferred to blend 0.1 to 40 % by mass of the total amount of the external skin preparation, and it is more preferred to blend 10 to 30 % by mass of the total amount of the external skin preparation.

Examples of dispersants include polyether-modified silicone, polyglycerin-modified silicone, carboxy-modified silicone, fatty acid added polyethylene glycol, fatty acid added polyglycerin, fatty acid, long-chain alcohol, and fatty acid sorbitan ester.

When dispersant is blended, it is preferred to blend 0.1 to 10 % by mass of the total amount of the external skin preparation, and it is more preferred to blend 0.5 to 5 % by mass of the total amount of the external skin preparation.

### ((c) Surfactant comprising one or more selected from polyoxyethylene hydrogenated castor oil, silicone-type surfactants, and sulfonic acid-type surfactants)

In the surfactant or component (c), it is necessary to contain at least one kind from polyoxyethylene hydrogenated castor oil, silicone-type surfactants, and sulfonic acid-type surfactants, and two or more kinds can be used in combination.

In the present invention, the blending quantity of the dispersant, which is used when powder and dispersant are blended in the (b) oil phase, is not included in the blending quantity of the (c) surfactant.

The addition mole number of oxyethylene groups in the polyoxyethylene hydrogenated castor oil is not limited in particular; however, it is preferably 30 to 100. If the addition mole number of oxyethylene groups is less than 30, the HLB is low (hydrophobicity is high) and it may not be suitable as an oil-in-water surfactant. If the addition mole number of oxyethylene groups exceeds 100, it may not function as an emulsifier.

Examples of silicone-type surfactants include organopolysiloxanes modified with a hydrophilic group, such as POE-modified organopolysiloxane, POE/POP-modified organopolysiloxane, POE sorbitan-modified organopolysiloxane, and POE glyceryl-modified organopolysiloxane.

Examples of sulfonic acid-type surfactants include sodium stearoyl methyltaurine, sodium lauryl sulfate, sulfated fatty acid monoglyceride sodium salt, sodium dodecylbenzenesulfonate, sodium kerylbenzenesulfonate, sodium monobutyl phenylphenol monosulfonate, sodium dibutyl phenylphenol disulfonate, and sodium dioctyl sulfosuccinate.

It is necessary to blend the surfactants, which are selected from the above-described polyoxyethylene hydrogenated castor oil, silicone-type surfactants, and sulfonic acid-type surfactants, in the amount of 0.01 mass % to 5 % by mass of the total amount of the external skin preparation and 30 % by mass or more of the total amount of surfactants. It is preferred to blend these surfactants in the amount of 50 % by mass or more of the total amount of surfactants, and it is especially preferred that these surfactants are blended in the amount of 100 % by mass.

If these surfactants are blended in the amount of less than 0.01 % by mass of the total amount of the external skin preparation, or blended in the amount of less than 30 % by mass of the total amount of surfactants, the emulsion stability may be poor. If the amount of these surfactants exceeds 5 % by mass of the total amount of the external skin preparation, texture may be poor.

Examples of surfactants other than polyoxyethylene hydrogenated castor oil, silicone-type surfactant, and sulfonic acid-type surfactant include polyoxyalkylene-type surfactants other than polyoxyethylene hydrogenated castor oil, amino acid-type surfactant, and fatty acid-type surfactant.

The oil-in-water external skin preparation of the present invention can be widely applied to cosmetics, pharmaceuticals, and quasi-drugs that have been applied to the skin. Examples include liquid, milky-liquid, or creamy products such as beauty essence, milky lotion, cream, milky lotion for daytime use, makeup base, liquid foundation, eyeliner, mascara, and hair gel, and products such as dermatological ointments.

### EXAMPLES

The present invention will be further described in the following examples. However, the invention is not limited by these examples. Unless otherwise specified, the blending quantity of each component will be expressed in mass %.

Prior to illustrating the examples, the methods for the evaluation tests used in the present invention will be explained.

### Evaluation (1): Stability over time

Samples were allowed to stand at 50 °C for 4 weeks, and then the appearance and viscosity were evaluated.
○: There is no change in appearance and viscosity; thus stability is good.
○Δ: There is slight change in appearance and/or viscosity; however, they are within a range of no problem.
Δ: There is some change in appearance and/or viscosity; thus attention is necessary.
Δ×: There is a change in appearance and/or viscosity; thus there is concern about stability.
×: There is big change in appearance and viscosity; thus there is lack of stability.

### Evaluation (2): Dispersion stability of powder

A sample was placed in a 50 ml sample tube, rotated at room temperature at a speed of 45 rpm for 4 hours, and then the degree of powder aggregation was visually evaluated.
○: There are no color streaks and aggregates.
○Δ: There are slight color streaks and aggregates; however, they are within a range of no problem.
Δ: There are some color streaks and aggregates.
Δ× : There are color streaks and aggregates.
×: There are numerous color streaks and aggregates.

### Evaluation (3): Rough-surface correction effect

The rough-surface correction effect when a sample was applied on the skin was evaluated by 10 professional panelists. The evaluation criteria are as follows.
○: 9 or more panelists answered that the sample had rough-surface correction effect.
○Δ: 7 or more to 9 or less panelists answered that the sample had rough-surface correction effect.
Δ: 5 or more to 7 or less panelists answered that the sample had rough-surface correction effect.
Δ×: 3 or more to 5 or less panelists answered that the sample had rough-surface correction effect.
×: 3 panelists or less answered that the sample had rough-surface correction effect.

### Evaluation (4): Texture

The texture when a sample was applied on the skin was evaluated by 10 professional panelists. The evaluation criteria are as follows.
○: 9 or more panelists answered that the texture was good.
○Δ: 7 or more to 9 or less panelists answered that the texture was good.
Δ: 5 or more to 7 or less panelists answered that the texture was good.
Δ×: 3 or more to 5 or less panelists answered that the texture was good.
×: 3 panelists or less answered that the texture was good.

Initially, the present inventors produced lotions (Test Examples 1-1 and 1-2) and oil-in-water foundations (Test Examples 1-3 to 1-7), in which various powder ingredients are blended in the aqueous phase, by the below-described production methods. Respective samples were evaluated, for the evaluation item (1) or (2), based on the above rating criteria. The results are shown in Table 1.

### • Production method of lotion

The respective lotions were obtained by sequentially adding each raw material into ion-exchanged water and mixing with stirring.

### • Production method of oil-in-water external skin preparation (foundation)

Dodecamethylcyclohexasiloxane, isododecane, cetyl isooctanoate, octyl methoxycinnnamate, amino-modified silicone, lauryl PEG-9 polydimethylsiloxyethyl dimethicone, isostearic acid, titanium oxide, colcothar, yellow iron oxide, and black iron oxide were mixed with stirring (oil phase part). Separately, ion-exchanged water, dynamite glycerin, 1,3-butylene glycol, carboxymethyl cellulose, dimethylacrylamide/Na acryloyldimethyltaurine crosspolymer, succinoglycan, methylparaben, phenoxyethanol, ethanol, trisodium edetate dihydrate, polyoxyethylene (60) hydrogenated castor oil were mixed with stirring, and then powder (spherical silica or polyurethane resin powder) was added and dispersed. The oil phase part was added to this and mixed with stirring to produce an oil-in-water external skin preparation.

**[Table 1]**

| | | Test Example | | | | | | |
|---|---|---|---|---|---|---|---|---|
| | | 1-1 | 1-2 | 1-3 | 1-4 | 1-5 | 1-6 | 1-7 |
| (a) | Spherical silica (*1) | 5 | - | - | 1 | 5 | - | - |
| | Polyurethane resin powder (*2) | - | 5 | - | - | - | 1 | 5 |
| | Ion-exchanged water | Q.S. | Q.S. | Q.S. | Q.S. | Q.S. | Q.S. | Q.S. |
| | Dynamite glycerin | 3 | 3 | 3 | 3 | 3 | 3 | 3 |
| | 1,3-butylene glycol | 5 | 5 | 5 | 5 | 5 | 5 | 5 |
| | Carboxymethyl cellulose | 0.15 | 0.15 | 0.15 | 0.15 | 0.15 | 0.15 | 0.15 |
| | Dimethylacrylamide/Na acryloyldimethyltaurine crosspolymer | 0.75 | 0.75 | 0.75 | 0.75 | 0.75 | 0.75 | 0.75 |
| | Succinoglycan | 0.07 | 0.07 | 0.07 | 0.07 | 0.07 | 0.07 | 0.07 |
| | Methylparaben | 0.17 | 0.17 | 0.17 | 0.17 | 0.17 | 0.17 | 0.17 |
| | Phenoxyethanol | 0.2 | 0.2 | 0.2 | 0.2 | 0.2 | 0.2 | 0.2 |
| | Ethanol | 2 | 2 | 2 | 2 | 2 | 2 | 2 |
| | Trisodium edetate dihydrate | 0.2 | 0.2 | 0.2 | 0.2 | 0.2 | 0.2 | 0.2 |
| (b) | Dodecamethylcyclohexasiloxane | - | - | 11.8 | 11.8 | 11.8 | 11.8 | 11.8 |
| | Isododecane | - | - | 1 | 1 | 1 | 1 | 1 |
| | Cetyl isooctanoate | - | - | 2 | 2 | 2 | 2 | 2 |
| | Octyl methoxycinnnamate | - | - | 3 | 3 | 3 | 3 | 3 |
| | Amino-modified silicone | - | - | 2 | 2 | 2 | 2 | 2 |
| | Lauryl PEG-9 polydimetylsiloxyethyl dimethicone | - | - | 0.5 | 0.5 | 0.5 | 0.5 | 0.5 |
| | Isostearic acid | - | - | 0.5 | 0.5 | 0.5 | 0.5 | 0.5 |
| | Silicone treated titanium oxide | - | - | 10 | 10 | 10 | 10 | 10 |
| | Silicone treated colcothar | - | - | 0.72 | 0.72 | 0.72 | 0.72 | 0.72 |
| | Silicone treated yellow iron oxide | - | - | 2.33 | 2.33 | 2.33 | 2.33 | 2.33 |
| | Silicone treated black iron oxide | - | - | 0.1 | 0.1 | 0.1 | 0.1 | 0.1 |
| (c) | Polyoxyethylene (60) hydrogenated castor oil | - | - | 1.2 | 1.2 | 1.2 | 1.2 | 1.2 |
| Evaluation (1): Stability over time | | ○ | ○ | - | - | - | - | - |
| Evaluation (2): Dispersion stability | | - | - | ○ | ○Δ | × | ○ | × |

| | | | | | | | | |
|---|---|---|---|---|---|---|---|---|
| *1: Sunsphere L-51 (manufactured by AGC Si-Tech. Co., Ltd.) *2: Plastic Powder D-400 (manufactured by TOSHIKI PIGMENT CO., LTD.) | | | | | | | | |

According to Table 1, the stability over time was excellent in both Test Examples 1-1 and 1-2, wherein 5 % by mass of a powder ingredient was blended in the aqueous composition.

On the other hand, the dispersion stability was good in Test Example 1-3, which is an oil-in-water external skin preparation wherein no powder ingredient was blended in the aqueous phase.

In Test Examples 1-4 and 1-5, which are oil-in-water external skin preparation wherein spherical silica was blended in the aqueous phase, the sample dispersion stability became poorer with an increase in its blending quantity.

In Test Examples 1-6 and 1-7, which are oil-in-water external skin preparation wherein polyurethane resin powder was blended in the aqueous phase, the dispersion stability also became poor when a large amount of the powder was blended.

Thus, even if a powder ingredient that is stably mixable in a water system is blended in the aqueous phase, which is the external phase of an emulsion system, the emulsion stability was confirmed to become poor especially when the powder ingredient is blended in a large amount.

Therefore, the present inventors have investigated the correlation between the powder in the aqueous phase and the emulsion stability.

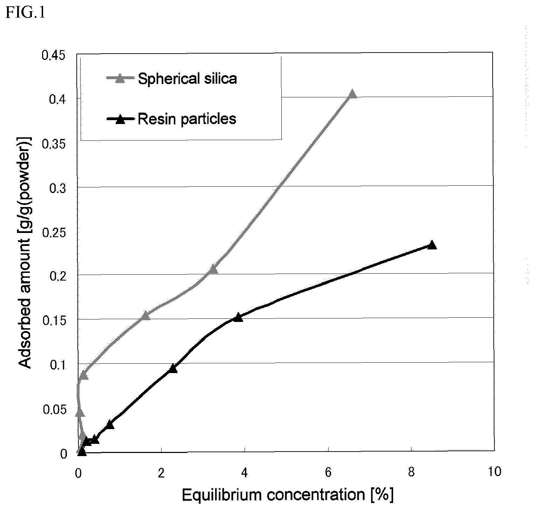

Into water, 10 % by mass of a powder and various concentrations (0.1, 0.3, 0.5, 1, 3, 5, and 10 % by mass) of the surfactant (polyoxyethylene (60) hydrogenated castor oil used in Table 1) were blended, and the adsorption isotherm was measured. As the powder, spherical silica and the resin particles produced by the below-described method were used for the investigation. The result is shown in Fig.1.

The structure of the monomer that was used in the production of resin particles and represented by the general formula (I) is shown below.

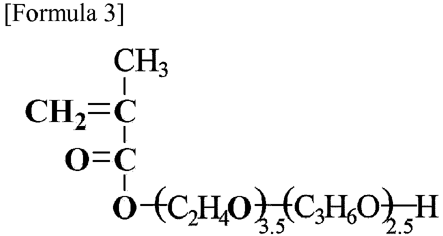

### • Synthetic method of resin particles

Into a 1 L reaction container with a stirrer and a thermometer, 500 g of water in which 1 g of sodium lauryl sulfate was dissolved was placed, and 120 g of calcium tertiary phosphate was dispersed in it. To this was added a beforehand-prepared mixed solution in which 63.4 g of poly(ethylene glycol-propylene glycol) monomethacrylate (product name: Blemmer 50PEP-300 (manufactured by NOF Corporation), l = about 3.5, m = about 2.5), represented by the above Formula 3, 1.0 g of benzoyl peroxide, and 1.5 g of azobisisobutyronitrile were dissolved in a mixture of 300 g of methyl methacrylate and 17 g of ethylene glycol dimethacrylate. By stirring with a T. K. homomixer, the droplet diameter was adjusted. Then, suspension polymerization was carried out by heating the reaction container to 50 °C with stirring. Subsequently, heat treatment was carried out at 100 °C for 2 hours, and resin particles with the average particle size of 7.9 µm were obtained.

According to Fig. 1, it is seen that the surfactant is adsorbed on the powder ingredient. Therefore, it was suggested that the adsorption of the surfactant on the powder ingredient may have harmful effects on emulsion stability.

In addition, it is seen that the amount of the adsorbed surfactant varies depending upon the kinds of powder ingredients. The amount of the adsorbed surfactant is small when the resin particles are blended compared with when spherical silica is blended.

Subsequently, the resin particles were blended in the aqueous phase of the oil-in-water emulsion system, and the stability was tested.

Oil-in-water foundations having the blending compositions shown in Table 2 below were produced by the above-described production method. Respective samples were evaluated, for the evaluation items (2) to (4), based on the above rating criteria. The results are shown in Table 2.

**[Table 2]**

| | | Test Example | | | |
|---|---|---|---|---|---|
| | | 1-4 | 1-5 | 2-1 | 2-2 |
| (a) | Spherical silica (*1) | 1 | 5 | - | - |
| | Resin particles of the present invention | - | - | 1 | 5 |
| | Ion-exchanged water | Q.S. | Q.S. | Q.S. | Q.S. |
| | Dynamite glycerin | 3 | 3 | 3 | 3 |
| | 1,3-butylene glycol | 5 | 5 | 5 | 5 |
| | Carboxymethyl cellulose | 0.15 | 0.15 | 0.15 | 0.15 |
| | Dimethylacrylamide/Na acryloyldimethyltaurine crosspolymer | 0.75 | 0.75 | 0.75 | 0.75 |
| | Succinoglycan | 0.07 | 0.07 | 0.07 | 0.07 |
| | Methylparaben | 0.17 | 0.17 | 0.17 | 0.17 |
| | Phenoxyethanol | 0.2 | 0.2 | 0.2 | 0.2 |
| | Ethanol | 2 | 2 | 2 | 2 |
| | Trisodium edetate dihydrate | 0.2 | 0.2 | 0.2 | 0.2 |
| (b) | Dodecamethylcyclohexasiloxane | 11.8 | 11.8 | 11.8 | 11.8 |
| | Isododecane | 1 | 1 | 1 | 1 |
| | Cetyl isooctanoate | 2 | 2 | 2 | 2 |
| | Octyl methoxycinnnamate | 3 | 3 | 3 | 3 |
| | Amino-modified silicone | 2 | 2 | 2 | 2 |
| | Lauryl PEG-9 polydimetylsiloxyethyl dimethicone | 0.5 | 0.5 | 0.5 | 0.5 |
| | Isostearic acid | 0.5 | 0.5 | 0.5 | 0.5 |
| | Silicone treated titanium oxide | 10 | 10 | 10 | 10 |
| | Silicone treated colcothar | 0.72 | 0.72 | 0.72 | 0.72 |
| | Silicone treated yellow iron oxide | 2.33 | 2.33 | 2.33 | 2.33 |
| | Silicone treated black iron oxide | 0.1 | 0.1 | 0.1 | 0.1 |
| (c) | Polyoxyethylene (60) hydrogenated castor oil | 1.2 | 1.2 | 1.2 | 1.2 |
| Evaluation (2): Dispersion stability | | ○Δ | × | ○ | ○ |
| Evaluation (3): Rough-surface correction effect | | Δ× | Δ | ○Δ | O |
| Evaluation (4): Texture | | Δ× | Δ× | ○Δ | ○Δ |

According to Table 2, the sample stability was good in Test Example 2-1, which is an oil-in-water external skin preparation wherein resin particles were blended in the aqueous phase.

The sample stability was also good in Test Example 2-2, wherein 5 % by mass of the resin particles were blended.

In Test Example 2-2, even when it was a normal oil-in-water external skin preparation without the use of a powder dispersion material in the oil phase, it was excellent in all the evaluations.

Thus, it was clarified that an oil-in-water external skin preparation excellent in stability can be obtained by blending the resin particles having a small amount of adsorbed surfactant (polyoxyethylene (60) hydrogenated castor oil) into the aqueous phase of the oil-in-water emulsion system, in which this surfactant was used.

In addition, it was clarified that the oil-in-water external skin preparation in which such resin particles were blended was also excellent in the rough-surface correction effect and texture.

Subsequently, an investigation was carried out concerning the amounts of various surfactants adsorbed on the resin particles.

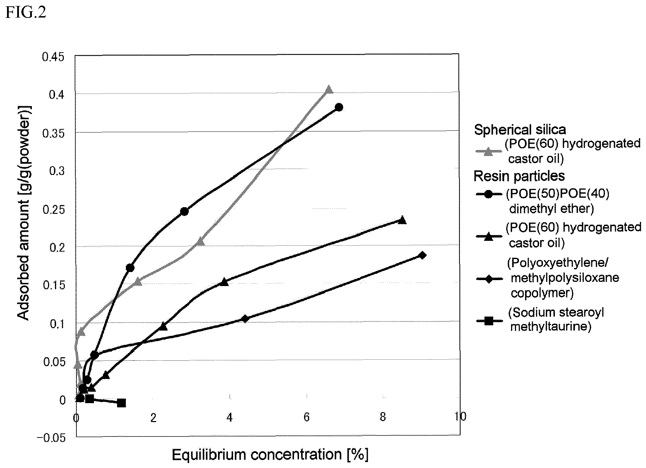

For the system in which 10 % by mass of the resin particles were blended in water, adsorption isotherms when various concentrations (0.3, 1, 3, 5, and 10 % by mass) of various surfactants were blended are shown in Fig. 2. In the results, the results of Fig. 1 are also included.

Because 3 % by mass or more of sodium stearoyl methyltaurine in Fig. 2 did not dissolve, the measurement was not possible. Therefore, the measurement was carried out at two concentrations, 0.3 % by mass and 1 % by mass. In addition, sodium stearate, which is a carboxylic acid-type surfactant, and sodium N-stearoyl-L-glutamate, which is an amino acid-type surfactant, were not soluble; thus the measurement could not be carried out.

According to Fig. 2, it is seen that the amount of adsorbed surfactant varies depending upon the kinds of surfactants even when the same resin particles were blended.

In particular, the amount of adsorbed POE(50)POP(40) dimethyl ether was very large irrespective of the concentration.

On the other hand, in addition to the case of polyoxyethylene (60) hydrogenated castor oil, when polyoxyethylene/methylpolysiloxane copolymer, which is a silicone-type surfactant, or sodium stearoyl methyltaurine, which is a sulfonic acid-type surfactant, was blended, the amount of adsorbed surfactant was small. Thus, it is understood that the formation of emulsion particles is not affected and that an emulsion composition of good emulsion stability can be obtained.

Thus, it was clarified that an external skin preparation excellent in stability and also excellent in the rough-surface correction effect and texture can be obtained by blending (c) a surfactant comprising one or more selected from polyoxyethylene hydrogenated castor oil, silicone-type surfactants, and sulfonic acid-type surfactants in the oil-in-water external skin preparation comprising (a) an aqueous phase containing resin particles and (b) an oil phase.

As a result of further investigation by the present inventors, it was found that it is necessary to satisfy the conditions that the blending quantity of one or more surfactants selected from (c) polyoxyethylene hydrogenated castor oil, silicone-type surfactants, and sulfonic acid-type surfactants is 0.01 to 5 % by mass of the total amount of the external skin preparation and 30 % by mass or more of the total amount of surfactants.

In here, formulation examples of the oil-in-water external skin preparation of the present invention will be illustrated. It is to be understood that the present invention is not limited by these formulation examples. All of the obtained oil-in-water external skin preparation was excellent in stability and also excellent in the rough-surface correction effect and texture. In the following formulation examples, the conventional method was used for production unless otherwise noted.

### Formulation Example 1: Oil-in-water makeup base

| | (% by mass) |
|---|---|
| Resin particles of the present invention | 5 |
| Ion-exchanged water | Q.S. |
| Dynamite glycerin | 5 |
| Dipropylene glycol | 2 |
| Carboxyvinyl polymer | 0.2 |
| Alkyl-modified carboxyvinyl polymer | 0.1 |
| Ethanol | 7 |
| Methylparaben | 0.15 |
| Potassium hydroxide | 1.2 |
| Citric acid | 0.01 |
| Sodium citrate | 0.09 |
| Trisodium edetate dihydrate | 0.03 |
| (Octylsilan/ quaternized ammonium chloride treated) silica coated titanium oxide | 4.5 |
| Ethylhexyl ethylhexanoate | 1.66 |
| Tripropylene glycol pivalate | 1.66 |
| Decamethylcyclopentasiloxane | 1.92 |
| Caprylyl methicone | 2 |
| Isododecane | 3 |
| Octyl methoxycinnamate | 4.07 |
| Octocrylene | 1.43 |
| Polyoxyethylene (60) hydrogenated castor oil | 0.5 |
| Polyoxyethylene (20) sorbitan monostearate | 0.5 |

### (Production method)

This was produced in the same way as the production method in Table 3.

### Formulation Example 2: Oil-in-water foundation

| | (% by mass) |
|---|---|
| Resin particles of the present invention | 10 |
| Ion-exchanged water | Q.S. |
| Dynamite glycerin | 1 |
| 1,3-butylene glycol | 4 |
| Carboxymethyl cellulose | 0.2 |
| Dimethylacrylamide/Na acryloyldimethyltaurine crosspolymer | 0.5 |
| Succinoglycan | 0.05 |
| Methylparaben | 0.2 |
| Phenoxyethanol | 0.2 |
| Ethanol | 2 |
| Trisodium edetate dihydrate | 0.2 |
| Dodecamethyl cyclohexasiloxane | 15 |
| Isododecane | 1 |
| Cetyl isooctanoate | 2 |
| Octyl methoxycinnamate | 5 |
| Amino-modified silicone | 2 |
| Lauryl PEG-9 polydimethylsiloxyethyl dimethicone | 0.5 |
| Isostearic acid | 0.5 |
| Silicone treated titanium oxide | 8 |
| Silicone treated colcothar | 0.7 |
| Silicone treated yellow iron oxide | 2 |
| Silicone treated black iron oxide | 0.1 |
| Polyoxyethylene (60) hydrogenated castor oil | 2 |

### (Production method)

This was produced in the same way as the production method in Table 2.

### Formulation Example 3: Cream

| | (% by mass) |
|---|---|
| Liquid paraffin | 3 |
| Vaseline | 1 |
| Dimethyl polysiloxane | 1 |
| Stearyl alcohol | 1.8 |
| Behenyl alcohol | 1.6 |
| Glycerin | 8 |
| Dipropylene glycol | 5 |
| Macademia nut oil | 2 |
| Hydrogenated oil | 3 |
| Squalane | 6 |
| Stearic acid | 2 |
| Colesteryl hydroxy stearate | 0.5 |
| Cetyl 2-ethylhexanoate | 4 |
| Polyoxyethylene hydrogenated castor oil | 0.5 |
| Self-emulsified glyceryl monostearate | 3 |
| Potassium hydroxide | 0.15 |
| Sodium hexametaphosphate | 0.05 |
| Trimethylglycine | 2 |
| Resin particles of the present invention | 5 |
| α-tocopherol 2-L-ascorbic acid phosphoric acid diester potassium salt | 1 |
| Tocopherol acetate | 0.1 |
| Sweet tea extract | 0.1 |
| Paraben | proper quantity |
| Trisodium edetate | 0.05 |
| 4-t-butyl-4'-methoxydibenzoyl methane | 0.05 |
| Glyceryl diparamethoxycinnamate mono-2-ethylhexanoate | 0.05 |
| Coloring agent | proper quantity |
| Carboxyvinyl polymer | 0.05 |
| Purified water | Q.S. |

### Formulation Example 4: Whitening essence

| | (% by mass) |
|---|---|
| Resin particles of the present invention | 1 |
| Vaseline | 2 |
| Dimethyl polysiloxane | 2 |
| Ethanol | 5 |
| Behenyl alcohol | 0.5 |
| Batyl alcohol | 0.2 |
| Glycerin | 7 |
| 1,3-buthylene glycol | 5 |
| Polyethyleneglycol 20000 | 0.5 |
| Jojoba oil | 3 |
| Squalane | 2 |
| Phytosterylhydroxystearate | 0.5 |
| Pentaerithrityl tetra2-ethylhexanoate | 1 |
| Polyoxyethylene hydrogenated castor oil | 1 |
| Potassium hydroxide | 0.1 |
| Sodium pyrosulfite | 0.01 |
| Sodium hexamethaphosphate | 0.05 |
| Stearyl glycyrrhetinate | 0.1 |
| Pantotenyl ethyl ether | 0.1 |
| Arbutin | 7 |
| Tranexamic acid | 1 |
| Tocopherol acetate | 0.1 |
| Sodium hyaluronate | 0.05 |
| p-hydroxybenzoate ester | proper quantity |
| Trisodium edetate | 0.05 |
| 4-t-butyl-4'-methoxydibenzoyl methane | 0.1 |
| Glyceryl diparamethoxycinnamate mono-2-ethylhexanoate | 0.1 |
| Yellow iron oxide | proper quantity |
| Xanthane gum | 0.1 |
| Carboxyvinyl polymer | 0.2 |
| Purified water | Q.S. |

### Formulation Example 5: Cleansing cream

| | (% by mass) |
|---|---|
| Liquid paraffin | 35 |
| Vaseline | 10 |
| Microcrystalline wax | 1 |
| Resin particles of the present invention | 5 |
| Cethanol | 2 |
| Batyl alcohol | 1 |
| Glycerin | 3 |
| Dipropylene glycol | 6 |
| Squalane | 6 |
| Stearic acid | 1 |
| Cetyl 2-ethylhexanoate | 10 |
| Polyoxyethylene hydrogenated castor oil | 1.5 |
| Polyoxyethylene hydrogenated castor oil pyroglutamate isostearate | 0.1 |
| Polyoxyethylene glycerin monostearate | 1 |
| Potassium hydroxide | 0.1 |
| Sodium metaphosphate | 0.02 |
| L-alginine | 0.1 |
| Paraben | proper quantity |
| Carboxyvinyl polymer | 0.05 |
| Purified water | Q.S. |
| Perfume | proper quantity |

### Formulation Example 6: Cleansing cream

| | (% by mass) |
|---|---|
| Dimethylpolysiloxane | 3 |
| Octamethylcyclotetrasiloxane | 20 |
| Ethyl alcohol | 5 |
| Resin particles of the present invention | 0.5 |
| Orange oil | 0.05 |
| Polyoxyethylene hydrogenated castor oil | 0.5 |
| Potassium hydroxide | proper quantity |
| Sodium hexametaphosphate | 0.01 |
| Hydroxypropyl-beta-cyclodextrin | 0.1 |
| Phellodendron bark extract | 0.1 |
| Phenoxyethanol | proper quantity |
| Carboxyvinyl polymer | 0.3 |
| Acrylate/methacrylate alkyl copolymer (PEMULEN TR-2) | 0.3 |
| Purified water | Q.S. |

### Formulation Example 7: Sunscreen cream

| | (% by mass) |
|---|---|
| Dimethylpolysiloxane | 5 |
| Decamethylcyclopentasiloxane | 25 |
| Trimethylsiloxysilicate | 5 |
| Polyoxyethylene/methylpolysiloxane copolymer | 2 |
| Dipropylene glycol | 5 |
| Fine particle zinc oxide (hydrophobized product, 60 nm) | 15 |
| Paraben | proper quantity |
| Phenoxyethanol | proper quantity |
| Trisodium edetate | proper quantity |
| 2-ethylhexyl p-methoxycinnamate | 7.5 |
| Dimethyl distearyl ammonium hectorite | 0.5 |
| Spherical alkyl polyacrylate powder | 5 |
| Resin particles of the present invention | 8 |
| Purified water | Q.S. |
| Perfume | proper quantity |

### Formulation Example 8: Milky lotion

| | (% by mass) |
|---|---|
| Vaseline | 5 |
| Behenyl alcohol | 0.5 |
| Batyl alcohol | 0.5 |
| Glycerin | 7 |
| 1,3-butylene glycol | 7 |
| 1,2-pentanediol | 1 |
| Xylitol | 3 |
| Polyethylene glycol 20000 | 2 |
| Hydrogenated oil | 2 |
| Jojoba oil | 2 |
| Squalane | 5 |
| Isostearic acid | 0.5 |
| Pentaerithrityl tetra 2-ethylhexanoate | 2 |
| Polyoxyethylene hydrogenated castor oil | 0.5 |
| Lauryl dimethyl amino acetate betaine | 0.4 |
| Potassium hydroxide | proper quantity |
| Sodium pyrosulfite | 0.01 |
| Sodium hexametaphosphate | 0.05 |
| Dipotassium glycyrrhizinate | 0.05 |
| Trimethylglycine | 3 |
| Arbutin | 3 |
| Yeast extract | 0.1 |
| Tocopherol acetate | 0.1 |
| Thiotaurine | 0.1 |
| Clara extract | 0.1 |
| Resin particles of the present invention | 1 |
| Colcothar | proper quantity |
| Quince seed extract | 0.1 |
| Carboxyvinyl polymer | 0.2 |
| Phenoxyethanol | proper quantity |
| Purified water | Q.S. |

### Formulation Example 9: Milky lotion

| | (% by mass) |
|---|---|
| Vaseline | 5 |
| Dimethylpolysiloxane | 2 |
| Behenyl alcohol | 0.6 |
| Batyl alcohol | 0.5 |
| Dipropylene glycol | 2 |
| 1,3-butylene glycol | 4 |
| Xylitol | 1 |
| Poly ethylenegly col 1500 | 1 |
| Resin particles of the present invention | 0.5 |
| Squalan | 5 |
| Glyceryl tri-2-ethylhexanoate | 2 |
| Polyoxyethylene hydrogenated castor oil | 0.5 |
| Dipotassium glycyrrhizinate | 0.1 |
| Yeast extract | 0.1 |
| Peony extract | 0.1 |
| Trisodium EDTA | 0.05 |
| Xanthane gum | 0.1 |
| Carboxyvinyl polymer | 0.15 |
| Purified water | Q.S. |
| Perfume | proper quantity |

### Formulation Example 10: Cleansing milky lotion

| | (% by mass) |
|---|---|
| Resin particles of the present invention | 1 |
| Liquid paraffin | 35 |
| Decamethylcyclopentasiloxane | 5 |
| Glycerin | 2 |
| Dipropylene glycol | 5 |
| 1,3-butylene glycol | 1 |
| Cetyl 2-ethylhexanoate | 8 |
| Polyoxyethylene hydrogenated castor oil | 2 |
| Sodium coconut oil fatty acid methyltaurine | 1 |
| Calcium chloride | 0.01 |
| Magnesium chloride | 0.01 |
| Phenoxyethanol | 0.2 |
| Trisodium edetate | 0.01 |
| Carboxyvinyl polymer | 0.15 |
| Purified water | Q.S. |
| Perfume | proper quantity |

### Formulation Example 11: Essence gel

| | (% by mass) |
|---|---|
| Resin particles of the present invention | 1.5 |
| Dimethylpolysiloxane | 5 |
| Glycerin | 2 |
| 1,3-butylene glycol | 5 |
| Polyethylene glycol 1500 | 3 |
| Polyethylene glycol 20000 | 3 |
| Cetyl octanoate | 3 |
| Citric acid | 0.01 |
| Sodium citrate | 0.1 |
| Sodium hexametaphosphate | 0.1 |
| Dipotassium glycyrrhizinate | 0.1 |
| Glucoside ascorbate | 2 |
| Tocopherol acetate | 0.1 |
| Scutellaria baicalensis root extract | 0.1 |
| Saxifraga sarmentosa extract | 0.1 |
| Trisodium edetate | 0.1 |
| Xanthane gum | 0.3 |
| Acrylate/methacrylate alkyl copolymer (PEMULEN TR-2) | 0.05 |
| Agar powder | 1.5 |
| Phenoxyethanol | proper quantity |
| Dibutyl hydroxytoluene | proper quantity |
| Purified water | Q.S. |

### (Production method)

By following the conventional method, a translucent emulsion composition was produced and then gelated by cooling to 30 °C or lower. When it was sufficiently solidified, the gel was crushed with a disper and microgels (average particle size: 70 µm) were obtained. Then, a gelatinous product was obtained by deaeration.

### Formulation Example 12: Oil-in-water emulsion foundation

| | (% by mass) |
|---|---|
| (1) Alkyl-modified silicone resin coated titanium oxide | 9 |
| (2) Alkyl-modified silicone resin coated ultrafine particle titanium oxide (40nm) | 5 |
| (3) Alkyl-modified silicone resin coated iron oxide (red) | 0.5 |
| (4) Alkyl-modified silicone resin coated iron oxide (yellow) | 1.5 |
| (5) Alkyl-modified silicone resin coated iron oxide (black) | 0.2 |
| (6) Polyoxyethylene-modified organo polysiloxane | 0.5 |
| (7) Decamethylpentacyclosiloxane | 5 |
| (8) Octyl p-methoxycinnamate | 5 |
| (9) Acrylic silicone | 4 |
| (10) PEG-100 hydrogenated castor oil | 2 |
| (11) Dynamite glycerin | 6 |
| (12) Xanthane gum | 0.1 |
| (13) Carboxymethyl cellulose | 0.3 |
| (14) Sodium acryloyldimethyltaurate/hydroxyethyl acrylate copolymer (SIMULGEL NS™ manufactured by SEPPIC) (Content: 35 to 40 mass %) | 1.5 |
| (15) Ethanol | 5 |
| (16) Resin particles of the present invention | 4 |
| (17) Ion-exchanged water | Q.S. |

### (Production method)

The components (1) to (9) were mixed and dispersed with a homo mixer and then added to the aqueous phase, wherein (10) to (17) were dissolved, while a homo mixer was being operated.

### Formulation Example 13: Makeup base

| | (% by mass) |
|---|---|
| Dimethylpolysiloxane 6mPas | 5 |
| Decamethylcyclopentasiloxane | Q.S. |
| Ethyl alcohol | 8 |
| Iron blue coated titanium mica | 0.5 |
| Methylsiloxane reticular copolymer | 5 |
| Cross-linked silicone powder (TREFIL E-506) | 15 |
| Resin particles of the present invention | 0.1 |
| Citric acid | 0.02 |
| Sodium citrate | 0.08 |
| Tocopherol acetate | 0.1 |
| Sigma-tocopherol | 0.1 |
| Melilot extract | 3 |
| Purified water | 1 |
| Poly (oxyethylene/oxypropylene) methyl polysiloxane copolymer | 5 |

### Formulation Example 14: Mascara

| | (% by mass) |
|---|---|
| Light isoparaffin | 6 |
| Dimethylpolysiloxane | 1 |
| Decamethylcyclopentasiloxane | 5 |
| Trimethylsiloxysilicate | 5 |
| Methylpolysiloxane emulsion | proper quantity |
| Isopropanol | 3 |
| 1,3-buthylene glycol | 6 |
| Polyoxyethylene hydrogenated castor oil | 1 |
| Saccharide fatty acid ester | 0.6 |
| Diglyceryl diisostearate | 1 |
| Sodium hydrogen carbonate | 0.01 |
| DL-α-tocopherol acetate | 0.1 |
| Acetylated sodium hyaluronate | 0.1 |
| p-oxybenzoate ester | proper quantity |
| Phenoxyethanol | 0.3 |
| Black iron oxide | 8 |
| Bentonite | 1 |
| Dimethyl distearyl ammonium hectorite | 4 |
| Polyvinyl alcohol | 4 |
| Alkyl acrylate copolymer emulsion | 12 |
| Polyvinyl acetate emulsion | 12 |
| Nylon fiber (1 to 2 mm) | 6 |
| Purified water | Q.S. |
| Resin particles of the present invention | 1.5 |
| Titanium oxide | 1 |
| Perfume | proper quantity |

### Formulation Example 15: Cream

| (Oil phase) | (% by mass) |
|---|---|
| Pentaerithrityl tetra2-ethyl hexanoate | 7.5 |
| Squalane | 5 |
| Dimethicone | 3 |
| Behenyl alcohol | 3 |
| Stearyl alcohol | 1 |
| Sodium stearoyl methyltaurine | 1 |

| (Water phase) | |
|---|---|
| Resin particles of the present invention | 13 |
| Glycerin | 10 |
| 1,3-butylene glycol | 8 |
| Carboxyvinyl polymer | 0.17 |
| Potassium hydroxide | 0.05 |
| Preservative | proper quantity |
| Purified water | Q.S. |

### (Production method)

To the aqueous phase at 70 °C, the oil phase preheated to 70 °C was added, dispersed uniformly with a homo mixer, and cooled to 30 °C to obtain the cream.

### Formulation Example 16: Cream

| | (% by mass) |
|---|---|
| Squalane | 5 |
| Pentaerithrityl tetraoctanoate | 8 |
| Propylene glycol diisostearate | 8 |
| Dimethicone | 3 |
| Cetanol | 3 |
| Stearyl alcohol | 2 |
| Polyoxyethylene (60) glyceryl isostearate | 2 |
| Resin particles of the present invention | 5 |
| Sodium stearoyl methyltaurine | 1 |
| Tranexamic acid | 1 |
| Glycerin | 10 |
| 1,3-butylene glycol | 7 |
| Preservative | proper quantity |
| Purified water | Q.S. |

## Claims

1. An oil-in-water external skin preparation, comprising
(a) an aqueous phase containing resin particles obtained from
(i) 100 parts by weight of a vinyl-type monomer, wherein the vinyl-type monomer(s) is selected from acrylic acid, methyl acrylate, ethyl acrylate, n-butyl acrylate, isobutyl acrylate, 2-ethylhexyl acrylate, methacrylic acid, n-butyl methacrylate, 2-ethylhexyl methacrylate, methyl methacrylate, ethyl methacrylate, isobutyl methacrylate, cyclohexyl methacrylate, benzyl methacrylate, isobornyl methacrylate, 2-hydroxyethyl methacrylate, 2-ethoxyethyl methacrylate, glycidyl methacrylate, tetrahydrofurfuryl methacrylate, diethylaminoethyl methacrylate, trifluoroethyl methacrylate, heptadecafluorodecyl methacrylate,
vinyl type monomers other than (meth)acrylic acid type monomers, namely styrene, p-methylstyrene, α-methylstyrene, vinyl acetate, alone or in combination of two or more,
crosslinked monomers, namely ethylene glycol di(meth)acrylate, diethylene glycol di(meth)acrylate, triethylene glycol di(meth)acrylate, tetradecaethylene glycol di(meth)acrylate, nonaethylene glycol di(meth)acrylate, tetradecaethylene glycol di(meth)acrylate, decaethylene glycol di(meth)acrylate, pentadecaethylene glycol di(meth)acrylate, 1,3-butylene glycol di(meth)acrylate, 1,4-butanediol di(meth)acrylate, 1,6-hexanediol di(meth)acrylate, glycerin di(meth)acrylate, trimethylol propane tri(meth)acrylate, pentaerithrityl tetra(meth)acrylate, diethylene glycol phthalate di(meth)acrylate, caprolactone-modified dipentaerithrityl hexa(meth)acrylate, caprolactone-modified hydroxypivalate ester neopentyl glycol diacrylate, polyester acrylate, urethane acrylate, divinyl benzene, and divinyl naphthalene, alone or in combination of two or more,
and (ii) 0.1 to 20 parts by weight of a monomer represented by the below general formula (I),
(b) an oil phase, and
(c) a surfactant comprising one or more selected from polyoxyethylene hydrogenated castor oil, silicone-type surfactants, and sulfonic acid-type surfactants, in the amount of 0.01 to 5 % by mass of the total amount of the external skin preparation and 30 % by mass or more of the total amount of surfactants, 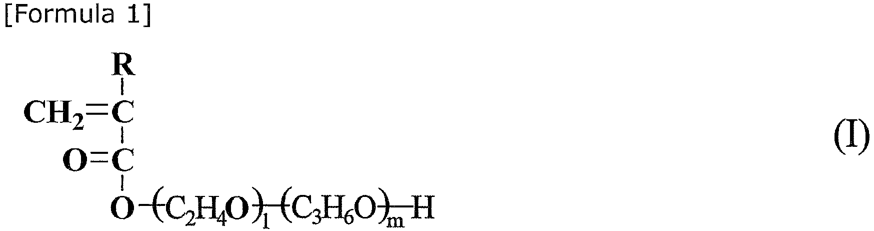
in the above formula, R represents a hydrogen atom or a methyl group, I represents 1 to 50, and m represents 1 to 50,
and wherein the oil-in-water external preparation is obtainable by blending resin particles in the aqueous phase and emulsifying with the surfactant.

2. The oil-in-water external skin preparation according to claim 1, wherein the resin particles are contained in the amount of 0.1 to 13 % by mass of the total amount of the external skin preparation.

3. The oil-in-water external skin preparation according to claim 1 or 2, wherein a powder and dispersant are contained in the oil phase.

4. The oil-in-water external skin preparation according to at least one of claims 1 to 3, wherein the vinyl-type monomers according to component (i) are used alone or in combination of two or more.

5. The oil-in-water external skin preparation according to at least one of claims 1 to 4, wherein the amount of the monomers selected from ethylene glycol di(meth)acrylate, diethylene glycol di(meth) acrylate, triethylene glycol di(meth)acrylate, tetradecaethylene glycol di(meth)acrylate, nonaethylene glycol di(meth)acrylate, tetradecaethylene glycol di(meth)acrylate, decaethylene glycol di(meth)acrylate, pentadecaethylene glycol di(meth)acrylate, 1,3-butylene glycol di(meth)acrylate, 1,4-butanediol di(meth)acrylate, 1,6-hexanediol di(meth)acrylate, glycerin di(meth)acrylate, trimethylol propane tri(meth)acrylate, pentaerithrityl tetra(meth)acrylate, diethylene glycol phthalate di(meth)acrylate, caprolactone-modified dipentaerithrityl hexa(meth)acrylate, caprolactone-modified hydroxypivalate ester neopentyl glycol diacrylate, polyester acrylate, urethane acrylate, divinyl benzene, and divinyl naphthalene, is 0.5 to 80% by weight of the total amount of vinyl-type monomer.

6. The oil-in-water external skin preparation according to at least one of claims 1 to 5, wherein a monomer represented by formula (I) is dissolved in vinyl-type monomer according to component (i) or dispersed in an aqueous medium.

7. The oil-in-water external skin preparation according to at least one of claims 1 to 6, wherein one or more thickener(s) selected from succinoglycan, xanthan gum, and acrylamide is blended in (a) water phase.

8. The oil-in-water external skin preparation according to at least one of claims 1 to 7, wherein the blending quantity of the (a) aqueous phase, namely aqueous components, is 50 to 90 % by mass of the total amount of the external skin preparation.

9. The oil-in-water external skin preparation according to at least one of claims 1 to 8, wherein the blending quantity of the (b) oil phase, namely oil components, is 1 to 40 % by mass of the total amount of the external skin preparation.

10. The oil-in-water external skin preparation according to at least one of claims 3 to 9, wherein the powder is blended to 0.1 to 40 % by mass of the total amount of the external skin preparation.

## Patentansprüche

1. Externes Öl-in-Wasser-Präparat für die Haut, umfassend:
(a) eine wässrige Phase enthaltend Harzpartikel und hergestellt aus
(i) 100 Gewichtsteile eines Monomers vom Vinyltyp, wobei das(die) Monomer(e) ausgewählt ist(sind) aus Acrylsäure, Methylacrylat, Ethylacrylat, n-Butylacrylat, Isobutylacrylat, 2-Ethylhexylacrylat, Methakrylsäure, n-Butylmethacrylat, 2-Ethylhexylmethacrylat, Methylmethacrylat, Ethylmethacrylat, Isobutylmethacrylat, Cyclohexylmethacrylat, Benzylmethacrylat, Isobornylmethacrylat, 2-Hydroxyethyl-Methacrylat, 2-Ethoxyethyl-Methacrylat, Glycidylmethacrylat, Tetrahydrofurfuyl-Methacrylat, Diethylaminoethyl-Methacrylat, Trifluoroethyl-Methacrylat, Heptadecafluorodecyl-Methacrylat,
Monomere vom Vinyltyp, mit Ausnahme von Monomeren vom (Meth)acyrlsäuretyp, und zwar Styrol, p-Methylstyrol, α-Methylstyrol, Vinylacetat, allein oder in Kombination von zwei oder mehreren, quervernetzte Monomere, und zwar Ethylenglycol-Di(meth)acrylat, Diethylenglycol-Di(meth)acrylat, Triethylenglycol-Di(meth)acrylat, Tetradecaethylenglycol-Di(meth)acrylat, Nonaethylenglycol-Di(meth)acrylat, Tetradecaethylenglycol-Di(meth)acrylat, Decaethylenglycol-Di(meth)acrylat, Pentadecaethylenglycol-Di(meth)acrylat, 1,3-Butylenglycol-Di(meth)acrylat, 1,4-butanedial-Di(meth)acrylat, 1,6-Hexandiol-Di(meth)acrylat, Glycerin-Di(meth)acrylat, Trimethylolpropan-Tri(meth)acrylat, Pentaerithrityl-Tetra(meth)acrylat, Diethyleneglycolphthalat-Di(meth)acrylat, caprolacton-modifiziertes Dipentaerithrityl-Hexa(meth)acrylat, caprolacton-modifiziertes Hydroxypivalat-Ester-Neopentylglycol-Diacrylate, Polyesteracrylat, Urethanacrylat, Divinylbenzen und Divinylnaphthalen, allein oder in Kombination von zwei oder mehreren, und
(ii) 0,1 bis 20 Gewichtsteile eines Monomers, dargestellt durch die nachstehende allgemeine Formel (I),
(b) eine Ölphase und
(c) ein Tensid, das eine oder mehrere der folgenden Substanzen enthält, die ausgewählt sind aus polyoxyethylen-hydrogenisiertem Rizinusöl, Tensiden vom Silikontyp und Tensiden vom Sulfonsäuretyp, in einer Menge von 0,01 bis 5 Massen-% der Gesamtmenge des externen Hautpräparats und 30 Massen-% oder mehr der Gesamtmenge an Tensiden, 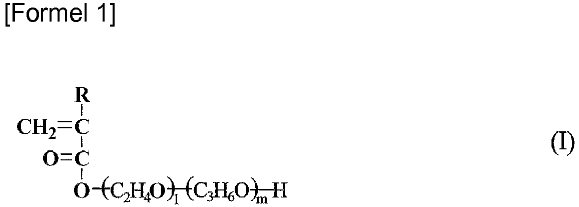
wobei in der vorstehenden Formel R ein Wasserstoff oder eine Methylgruppe darstellt, l 1 bis 50 darstellt und m 1 bis 50 darstellt und wobei sich das externe Öl-in-Wasser-Präparat erhalten lässt durch das Mischen von Harzpartikeln in der wässrigen Phase und das Emulgieren mit dem Tensid.

2. Externes Öl-in-Wasser-Präparat für die Haut gemäß Anspruch 1, wobei die Harzpartikel enthalten sind in der Menge von 0,1 bis 13 Massen-% der Gesamtmenge des externen Hautpräparats.

3. Externes Öl-in-Wasser-Präparat für die Haut gemäß Anspruch 1 oder 2, wobei ein Pulver und ein Dispergiermittel in der Ölphase enthalten sind.

4. Externes Öl-in-Wasser-Präparat für die Haut gemäß wenigstens einem der Ansprüche 1 bis 3, wobei die Monomere vom Vinyltyp gemäß Komponente (i) allein oder in Kombination von einem oder mehreren verwendet werden.

5. Externes Öl-in-Wasser-Präparat für die Haut gemäß wenigstens einem der Ansprüche 1 bis 4, wobei die Menge an Monomeren, die ausgewählt sind aus Ethylenglycol-Di(meth)acrylat, Diethylenglycol-Di(meth)acrylat, Triethylenglycol-Di(meth)acrylat, Tetradecaethylenglycol-Di(meth)acrylat, Nonaethylenglycol-Di(meth)acrylat, Tetradecaethylenglycol-Di(meth)acrylat, Decaethylenglycol-Di(meth)acrylat, Pentadecaethylenglycol-Di(meth)acrylat, 1,3-Butylenglycol-Di(meth)acrylat, 1,4-butanedial-Di(meth)acrylat, 1,6-Hexandiol-Di(meth)acrylat, Glycerin-Di(meth)acrylat, Trimethylolpropan-Tri(meth)acrylat, Pentaerithrityl-Tetra(meth)acrylat, Diethyleneglycolphthalat-Di(meth)acrylat, caprolacton-modifiziertes Dipentaerithrityl-Hexa(meth)acrylat, caprolacton-modifiziertes Hydroxypivalat-Ester-Neopentylglycol-Diacrylate, Polyesteracrylat, Urethanacrylat, Divinylbenzen und Divinylnaphthalen 0,5 bis 80 Gew.-% der Gesamtmenge des Monomers vom Vinyltyp beträgt.

6. Externes Öl-in-Wasser-Präparat für die Haut gemäß wenigstens einem der Ansprüche 1 bis 5, wobei ein durch Formel (I) dargestelltes Monomer in einem Monomer vom Vinyltyp gemäß Komponente (i) aufgelöst wird oder in einem wässrigen Medium dispergiert wird.

7. Externes Öl-in-Wasser-Präparat für die Haut gemäß wenigstens einem der Ansprüche 1 bis 6, wobei ein oder mehrere Verdickungsmittel, die ausgewählt sind aus Succinoglykan, Xanthangummi und Acrylamid, in (a) wässriger Phase gemischt werden.

8. Externes Öl-in-Wasser-Präparat für die Haut gemäß wenigstens einem der Ansprüche 1 bis 3, wobei die Mischmenge der (a) wässrigen Phase, und zwar der wässrigen Komponenten, 50 bis 90 Massen-% der Gesamtmenge des externen Hautpräparats beträgt.

9. Externes Öl-in-Wasser-Präparat für die Haut gemäß wenigstens einem der Ansprüche 1 bis 8, wobei die Mischmenge der (b) Ölphase, und zwar der Ölkomponenten, 1 bis 40 Massen-% der Gesamtmenge des externen Hautpräparats beträgt.

10. Externes Öl-in-Wasser-Präparat für die Haut gemäß wenigstens einem der Ansprüche 3 bis 9, wobei das Pulver zum Erreichen von 0,1 bis 40 Massen-% der Gesamtmenge des externen Hauptpräparats gemischt wird.

## Revendications

1. Préparation externe de type huile-dans-eau pour les soins de la peau, comprenant
(a) une phase aqueuse contenant des particules de résine obténues à partir de
(i) 100 parties en poids d'un monomère vinylique, le(les) monomère(s) vinylique(s) étant choisis parmi l'acide acrylique, l'acrylate de méthyle, l'acrylate d'éthyle, l'acrylate de n-butyle, l'acrylate d'isobutyle, l'acrylate de 2-éthylhexyle, l'acide méthacrylique, le méthacrylate de n-butyle, le méthacrylate de 2-éthylhexyle, le méthacrylate de méthyle, le méthacrylate de éthyle, le méthacrylate d'isobutyle, le méthacrylate de cyclohexyle, le méthacrylate de benzyle, le méthacrylate d'isobornyle, le méthacrylate de 2-hydroxyéthyle, le méthacrylate de 2-éthoxyéthyle, le méthacrylate de glycidyle, le méthacrylate de tetrahydrofurfuryle, le méthacrylate de diéthylaminoéthyle, le méthacrylate de trifluoroéthyle, le méthacrylate de heptadecafluorodécyle,
de monomères vinyliques de type autre que les monomères de type (méth)acrylique,
à savoir le styrène, le p-méthylstyrène, l'a-méthylstyrène, l'acétate de vinyle, seuls ou en combinaison de deux ou plusieurs,
de monomères réticulés, à savoir le di(méth)acrylate d'éthylèneglycol, le di(méth)acrylate de diéthylèneglycol, le di(méth)acrylate de triéthylèneglycol, le di(méth)acrylate de tetradécaéthylèneglycol, le di(méth)acrylate de nonaéthylèneglycol, le di(méth)acrylate de tetradécaéthylèneglycol, le di(méth)acrylate de décaéthylèneglycol, le di(méth)acrylate de pentadécaéthylèneglycol, le di(méth)acrylate de 1,3-butylèneglycol, le di(méth)acrylate de 1,4-butanediol, le di(méth)acrylate de 1,6-hexanediol, le di(méth)acrylate de glycérine, le tri(méthacrylate) de trimétholylpropane, le tetra(méthyl)acrylate de pentaérithrityle, le di(méth)acrylate de phthalate de diéthylèneglycol, le hexa(méth)acrylate de dipentaérithryle mofifié par caprolactone, le diacrylate de néopentylglycol d'éster d'hydroxypivalate modifié par caprolactone, l'acrylate de polyéster, l'uréthane acrylate, le divinylbenzène et le divinylnaphthalène, seuls ou en combinaison de deux ou plusieurs, et
(ii) 0,1 à 20 parties en poids d'un monomère représenté par la formule générale (l) ci-dessous,
(b) une phase huileuse et
(c) un tensioactif comprenant un ou plusieurs choisis parmi l'huile de ricin hydrogénée de polyoxyéthylène, les tensioactifs de type silicone et les tensioactifs de type acide sulfonique d'un montant de 0,01 à 5% en poids du montant total de tensioactifs, 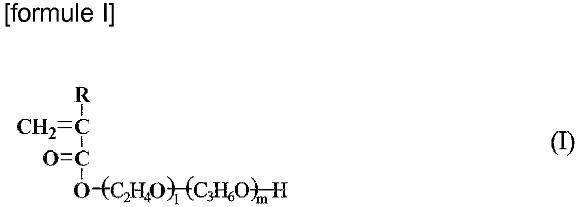
dans laquelle R représente un atome d'hydrogène ou un groupe méthyle, l représente 1 à 50 et m représente 1 à 50,
et dans laquelle la préparation externe de type huile-dans-eau pour les soins de la peau peut être obtenue en mélangeant des particules de résine dans la phase aqueuse et en émulsifiant avec le tensioactif.

2. Préparation externe de type huile-dans-eau pour les soins de la peau selon la revendication 1, dans laquelle les particules de résine sont contenues dans la quantité de 0,1 à 13% en poids de la quantité totale de la préparation externe pour les soins de la peau.

3. Préparation externe de type huile-dans-eau pour les soins de la peau selon la revendication 1 ou 2, dans laquelle une poudre et un agent de dispersion sont contenus dans la phase huileuse.

4. Préparation externe de type huile-dans-eau pour les soins de la peau selon au moins un des revendications 1 à 3, dans laquelle les monomères de type vinyle selon le composant (i) sont utilisés seuls ou en combinaison deux ou plusieurs.

5. Préparation externe de type huile-dans-eau pour les soins de la peau selon au moins un des revendications 1 à 4, dans laquelle la quantité de monomères choisi parmi le di(méth)acrylate d'éthylèneglycol, le di(méth)acrylate de diéthylèneglycol, le di(méth)acrylate de triéthylèneglycol, le di(méth)acrylate de tetradécaéthylèneglycol, le di(méth)acrylate de nonaéthylèneglycol, le di(méth)acrylate de tetradécaéthylèneglycol, le di(méth)acrylate de décaéthylèneglycol, le di(méth)acrylate de pentadécaéthylèneglycol, le di(méth)acrylate de 1,3-butylèneglycol, le di(méth)acrylate de 1,4-butanediol, le di(méth)acrylate de 1,6-hexanediol, le di(méth)acrylate de glycérine, le tri(méthacrylate) de trimétholylpropane, le tetra(méthyl)acrylate de pentaérithrityle, le di(méth)acrylate de phthalate de diéthylèneglycol, le hexa(méth)acrylate de dipentaérithryle mofifié par caprolactone, le diacrylate de néopentylglycol d'éster d'hydroxypivalate modifié par caprolactone, l'acrylate de polyéster, l'uréthane acrylate, le divinylbenzène et le divinylnaphthalène est 0,5 à 80% en poids de la quantité totale du monomère de type vinyle.

6. Préparation externe de type huile-dans-eau pour les soins de la peau selon au moins un des revendications 1 à 5, dans laquelle un monomère représenté par la formule (I) se dissoudre dans un monomère de type vinyle selon le composant (i) ou est dispersée dans un milieu aqueux.

7. Préparation externe de type huile-dans-eau pour les soins de la peau selon au moins un des revendications 1 à 6, dans laquelle un ou plusieurs épaisseurs choisi(s) parmi le succinoglycane, la gomme xanthane et l'acrylamide est(sont) mélangé(s) dans (a) une phase aqueuse.

8. Préparation externe de type huile-dans-eau pour les soins de la peau selon au moins un des revendications 1 à 7, dans laquelle la quantité de mélange de (a) la phase aqueuse, à savoir de composants aqueux, est 50 à 90% en poids de la quantité totale de la préparation externe de peau.

9. Préparation externe de type huile-dans-eau pour les soins de la peau selon au moins un des revendications 1 à 8, dans laquelle la quantité de mélange de (b) la phase huileuse, à savoir de composants huileux, est 1 á 40% en poids de la quantité totale de la préparation externe de peau.

10. Préparation externe de type huile-dans-eau pour les soins de la peau selon au moins un des revendications 3 à 9, dans laquelle la poudre est mélangée pour obtenir 0,1 à 40% en poids de la quantité totale de la préparation externe de peau.
